# EUROPEAN PATENT APPLICATION

(11) **EP 4 067 489 A1**
(43) Date of publication of application: **05.10.2022**
(21) Application number: 20894697.0
(22) Date of filing: 27.11.2020
(51) Int. Cl.: C12N 15/113, A61K 31/7125, A61K 48/00, A61P 43/00, C07H 21/04

(54) **METHOD FOR REDUCING TOXICITY OF ANTISENSE NUCLEIC ACIDS**

(30) Priority: 27.11.2019 JP 2019214766
(71) Applicant: Tokyo Institute of Technology, Tokyo 152-8550 (JP)
(72) Inventor: SEIO, Kohji, Tokyo 152-8550 (JP); MASAKI, Yoshiaki, Tokyo 152-8550 (JP); TOMORI, Takahito, Tokyo 152-8550 (JP)
(74) Representative: Vossius & Partner Patentanwälte Rechtsanwälte mbB
(86) International application number: PCT/JP2020/044370
(87) International publication number: WO 2021/107143

(57) **Abstract**

The purpose of the present invention is to reduce the strong hepatotoxicity of antisense nucleic acids that incorporate an artificial nucleobase (e.g., LNA). The present invention provides a bridged antisense nucleic acid for which antisense nucleic acid-induced toxicity is reduced by the supplemental addition to the wing region of a specific artificial nucleobase (e.g., MCA), while retaining the sequence of the antisense nucleic acid and the number of artificial nucleobases (e.g., LNA). The present invention also provides an antisense nucleic acid drug having a remarkably-reduced hepatotoxicity.

## Description

### FIELD

The present invention relates to antisense nucleic acid having activity of inhibiting and regulating expression of a target gene by an antisense effect, being bridged antisense nucleic acid whereby the toxicity of the antisense nucleic acid is reduced. More specifically, the invention relates to bridged antisense nucleic acid, whereby supplemental addition and/or insertion of 2'-modified nucleic acid to the nucleotide sequence of bridged nucleic acid allows the antisense effect to be maintained or augmented and allows side-effects to be reduced.

### BACKGROUND

Amid the recent ongoing development of oligonucleotides to be used as pharmaceuticals known as nucleic acid drugs, development is particularly active in the area of nucleic acid drugs utilizing antisense methods, from the viewpoint of greater selectivity of target genes. An antisense method is a method in which an oligonucleotide (antisense oligonucleotide, ASO) complementary to a partial sequence of target gene mRNA (sense strand) is introduced into cells to selectively inhibit or regulate expression of the protein encoded by the target gene.

Most pipelines of ASO clinical trials use PS-type ASOs. A PS-type ASO is phosphorothioated (PS), meaning that one of the oxygen atoms of the phosphate group in a phosphodiester bond which is the naturally-occurring bond between nucleic acids, is replaced with a sulfur atom. PS results in improvements from the viewpoint of nuclease resistance (NPL 1) and uptake into cells (NPL 2), but it is also known to cause various side-effects such as hepatotoxicity (NPL 3).

One typical PS-type ASO is mipomersen sodium (trade name: Kynamro), developed by Ionis Pharmaceuticals as an anti-cholesterolemia therapeutic drug having a Gapmer structure, which is known as a "second-generation nucleic acid structure". While being the first systemically administered nucleic acid drug to be approved in the U.S. in 2013, and indicated for homozygous familial hypercholesterolemia, it has been found to have several side-effects such as hepatotoxicity, similar to PS-type ASOs. It has failed to gain approval in Europe because of its side-effects (NPL 4).

Other ASOs that are known include bridged artificial nucleic acids such as 2',4'-BNA (LNA), cEt, ENA and AmNA, which have increased binding affinity for target RNA. These are all characterized by being 2',4'-modified, and are expected to improve the drug effect of ASOs due to their high binding strength for target RNA, increased activity, and shorter ASOs (NPL 5).

It is recently being attempted to reduce the hepatotoxicity of bridged antisense nucleic acids by adding chemical modifications to the gap portions of the structure (PTL 1).

### [CITATION LIST]

### [PATENT LITERATURE]

[PTL 1] International Patent Publication No. 2018/155450 [NON PATENT LITERATURE]

[NPL 1] Stein, C. A., et al., Nucleic Acids Res., 16, 3209-3221 (1988)
[NPL 2] Zhao, Q., et al., Antisense Res. Dev., 3, 53-66(2009), doi: 10.1089/ard.1993.3.53
[NPL 3] Agrawal S., Trends Biotechnol., 14, 376-87 (1996)
[NPL 4] European Medicines Agency, EMA/792736/2012, EMEA/H/C/002429, http://www.buenavistainv.com/dbimages/Isis%20Presentation.pdf
[NPL 5] Obika, S., Kasahara, Y., Nippon Yakurigaku Zasshi 148,100-104 (2016)

### SUMMARY

### [TECHNICAL PROBLEM]

Thus, while antisense nucleic acids exhibiting clinical effects are useful when incorporating artificial nucleic acid bases (LNA), they usually exhibit strong hepatotoxicity. Possible methods for avoiding such toxicity include reducing the number of LNAs introduced or altering the sequence of the antisense nucleic acid itself, but such methods often lower the pharmaceutical activity. It is therefore an object of the present invention to provide a bridged antisense nucleic acid and drug having reduced hepatotoxicity while maintaining or augmenting the antisense nucleic acid activity.

### [SOLUTION TO PROBLEM]

As a result of conducting much research with the aim of providing such an antisense nucleic acid drug, the present inventors have completed this invention upon finding that it is possible to reduce the toxicity of antisense nucleic acid by supplemental addition and/or insertion of a specific artificial nucleic acid base while maintaining the antisense nucleic acid sequence and number of LNAs.

Specifically, the present invention provides the following.
[1] Bridged antisense nucleic acid comprising a gap region consisting of deoxyribonucleic acid of 5 to 15 bases and a wing region consisting of two to ten 2',4'-modified nucleic acids at each of the 5' and 3'-ends of the gap region, wherein an additional one to four 2'-modified nucleic acids are added and/or inserted in at least one wing region.
[2] The bridged antisense nucleic acid according to [1], wherein the 2'-modified nucleic acid has the following structural formula: 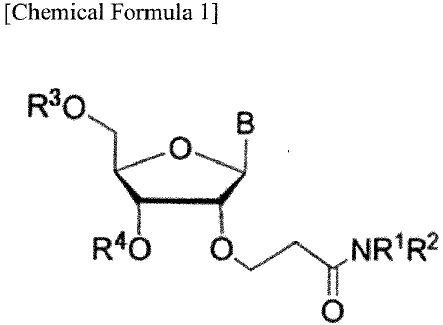 [wherein
   R¹ and R² are each independently selected from the group consisting of H, substituted or unsubstituted alkyl groups, substituted or unsubstituted aralkyl groups, substituted or unsubstituted alkenyl groups, substituted or unsubstituted alkynyl groups and substituted or unsubstituted aryl groups;
   R³ is H or the structure: 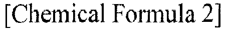 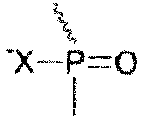 (wherein 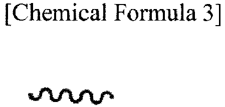 is the bonding point with the adjacent nucleic acid, or OH; and
      X is S or O);
      R⁴ is H or the bonding point with the adjacent nucleic acid; and
      B represents a nucleobase residue optionally having a protecting group or a modifying group].
[3] The bridged antisense nucleic acid according to [2], wherein R¹ is H and R² is a methyl group.
[4] The bridged antisense nucleic acid according to any one of [1] to [3], wherein one or two 2'-modified nucleic acids are added and/or inserted in each wing region.
[5] The bridged antisense nucleic acid according to any one of [1] to [4], wherein the 2',4'-modified nucleic acid is selected from the group consisting of the following: 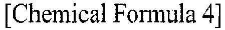 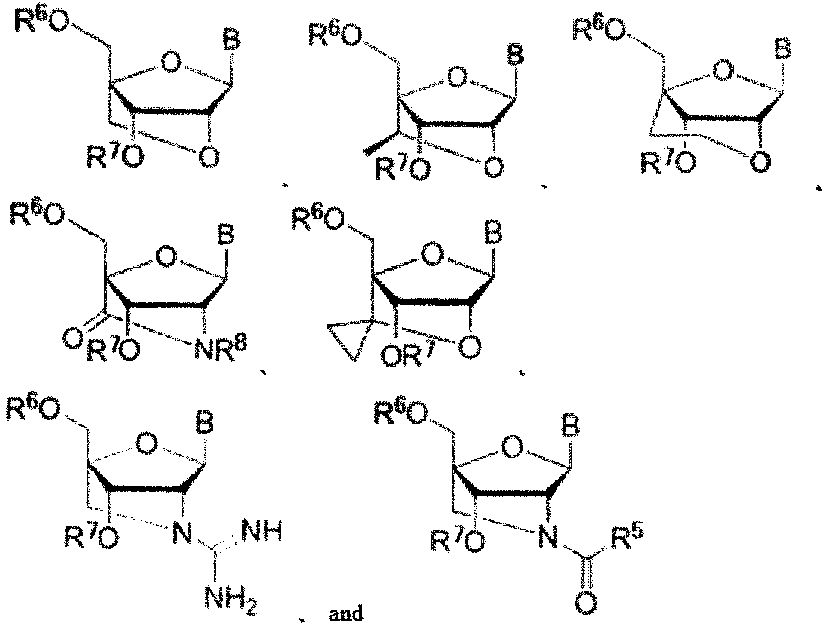 [wherein
   R⁵ and R⁸ are each independently selected from the group consisting of H, substituted or unsubstituted alkyl groups, substituted or unsubstituted aralkyl groups, substituted or unsubstituted alkenyl groups, substituted or unsubstituted alkynyl groups and substituted or unsubstituted aryl groups;
   R⁶ is H or the structure: 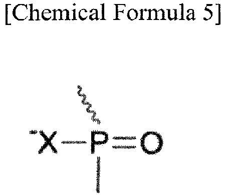 (wherein 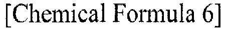 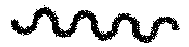 is the bonding point with the adjacent nucleic acid, or OH; and
      X is S or O);
      R⁷ is H or the bonding point with the adjacent nucleic acid; and
      B represents a nucleobase residue optionally having a protecting group or a modifying group].
[6] The bridged antisense nucleic acid according to any one of [1] to [5], which comprises two to four 2',4'-modified nucleic acids.
[7] The bridged antisense nucleic acid according to any one of [1] to [6], wherein X is a sulfur atom.
[8] The bridged antisense nucleic acid according to any one of [1] to [7], wherein the deoxyribonucleic acid has a base length of 8 to 10.
[9] An antisense nucleic acid drug with reduced toxicity by antisense nucleic acid, comprising bridged antisense nucleic acid according to any one of [1] to [8].

### [ADVANTAGEOUS EFFECTS OF INVENTION]

According to the invention it is possible to reduce the toxicity produced by conventional bridged antisense nucleic acids and to provide antisense nucleic acids with further increased drug effects, by adding and/or inserting 2'-modified nucleic acid into the wing regions consisting of bridged nucleic acid, in bridged antisense nucleic acid.

### BRIEF DESCRIPTION OF DRAWINGS

Fig. 1 shows the results of purification and analysis of synthesized GTCTGTGCATCTCTCC (SEQ ID NO: 1) by reverse-phase HPLC.
Fig. 2 shows the results of purification and analysis of synthesized GTcTGTGCATCTCtCC (SEQ ID NO: 2) by reverse-phase HPLC.
Fig. 3 shows the results of purification and analysis of synthesized GTCtGTGCATCTcTCC (SEQ ID NO: 3) by reverse-phase HPLC.
Fig. 4 shows the results of purification and analysis of synthesized cGTCTGTGCATCTCTCCt (SEQ ID NO: 4) by reverse-phase HPLC.
Fig. 5 shows the results of purification and analysis of synthesized CGtCTGTGCATCTCtCCT (SEQ ID NO: 5) by reverse-phase HPLC.

### DESCRIPTION OF EMBODIMENTS

The present invention relates to bridged antisense nucleic acid with reduced toxicity comprising 2'-modified nucleic acid, and to a therapeutic agent comprising the bridged antisense nucleic acid. The present invention will now be explained in greater detail.

### (1) Bridged antisense nucleic acid

The bridged antisense nucleic acid of the invention comprises a gap region consisting of deoxyribonucleic acid and a wing region consisting of 2',4'-modified nucleic acids at each of the 5' and 3'-ends of the gap region, wherein additional 2'-modified nucleic acids are added and/or inserted in each wing region.

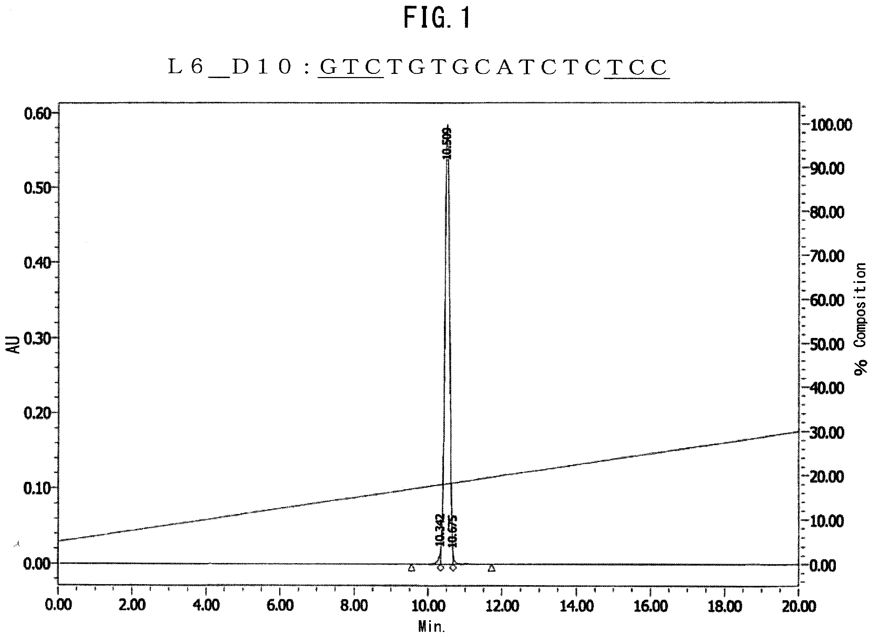

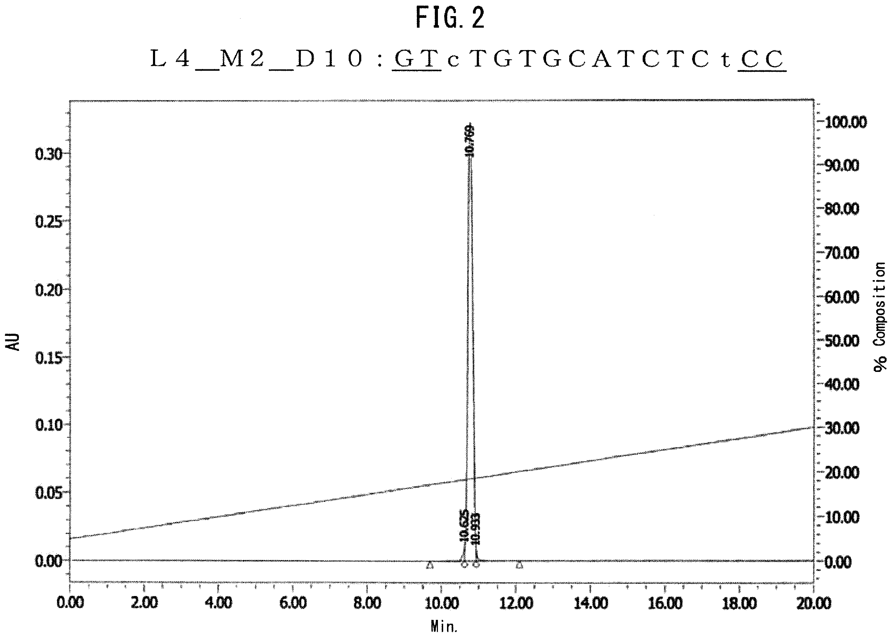

The structure of the bridged antisense nucleic acid of the invention is that of conventional antisense nucleic acid ("L6 -D10") having a structure comprising a gap region consisting of a nucleotide sequence complementary to the nucleotide sequence of the target nucleic acid (a region consisting of "DNA", with no limitation on the form of bonding between the nucleic acids, which may be phosphorothioate bonding or phosphodiester bonding, and preferably phosphorothioate bonding) (of any length but preferably 8 to 10 bases), and wing regions situated on either end of the gap region (the two regions consisting of "LNA" in Fig. 1, for example, are underlined), with 2'-modified nucleic acid ("MCE") supplementally added and/or inserted at the ends or interior of the wing regions (MCE is shown in lowercase in Fig. 2).

The wing regions of the bridged antisense nucleic acid of the invention comprise or consist of 2',4'-modified nucleic acid, and the types and numbers of 2',4'-modified nucleic acid are not restricted so long as they contribute to the effect of reducing toxicity of the bridged antisense nucleic acid (see W. Brad Wan and Punit P. Seth, J. Med. Chem. 2016, 59, 9645-9667, for example). Moreover, the form of bonding between the nucleic acids is not restricted and may be phosphorothioate bonding or phosphodiester bonding, although phosphorothioate bonding is preferred. The "phosphorothioate bonding" form has one of the oxygen atoms of a phosphate group in a phosphodiester bond, which is the naturally-occurring bond between nucleic acids, replaced with a sulfur atom, thus being phosphorothioated (PS).

According to the invention, the 2',4'-modified nucleic acid is not restricted and may have the following structural formula: 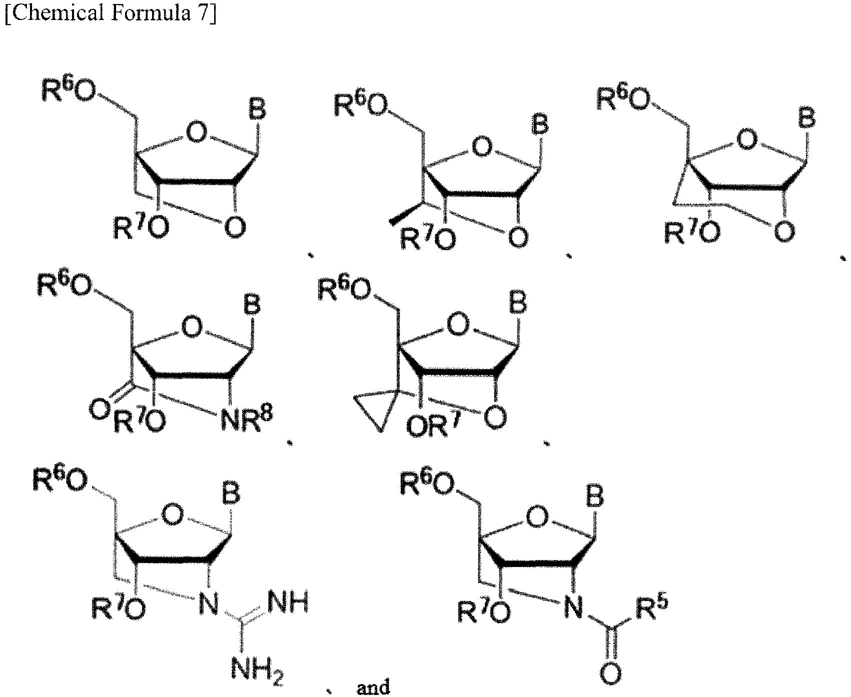 [wherein
R⁵ and R⁸ are each independently selected from the group consisting of H, substituted or unsubstituted alkyl groups, substituted or unsubstituted aralkyl groups, substituted or unsubstituted alkenyl groups, substituted or unsubstituted alkynyl groups and substituted or unsubstituted aryl groups;
R⁶ is H or the structure: 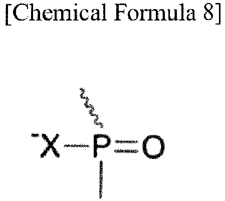 (wherein 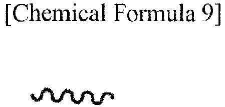 is the bonding point with the adjacent nucleic acid, or OH; and
   X is S or O);
   R⁷ is H or the bonding point with the adjacent nucleic acid; and
   B represents a nucleobase residue optionally having a protecting group or a modifying group]. B represents a nucleobase residue optionally having a protecting group or a modifying group. Examples of nucleobases include naturally-occurring nucleobases such as adenine, cytosine, guanine, uracil, thymine and 5-methylcytosine, and non-naturally occurring nucleobases such as 2-thiouracil, 2-thiothymine, 2-thiocytosine, 2-thio-5-methylcytosine, 2,4-diaminopurine, 6-thioguanine, uracil-5-yl, 7-deazaguanine, 7-deazaadenine, 3-deazaguanine and 3-deazaadenine. Protecting groups or modifying groups to be introduced onto nucleobases may be one or two acyl groups protecting the amino group, or they may be amidine-type protecting groups. The oxygen atom of a keto group may be tautomerized to a hydroxyl group, and may be protected with an alkyl group such as a cyanoethyl, 2-(4-nitrophenyl)ethyl, 2-nitrobenzyl or acyloxymethyl group, or an acyl group such as a diphenylcarbamoyl group. For a uracil base or thymine base, the N3 position may be protected with an alkyl or acyl group. The carbon atoms of naturally-occurring nucleobases and non-naturally occurring nucleobases may have substituents including amino, halogen, acyl, cyano, alkyl, alkenyl, alkynyl, aryl, aralkyl, alkoxy, alkylthio, acylamino, carbamoyl, carboxy or acyloxy groups, which may further have fluorescent functional groups, biotinyl groups, amino groups or spin labels.

As is publicly known, the sugar portions of natural nucleic acid (DNA or RNA) have a 5-membered ring consisting of four carbon atoms and one oxygen atom, the sugar portion being in either the N- or S-conformation. Since the target mRNA of ASO primarily adopts an A-type helical structure with the sugar chains in the N-conformation, it is important for ASO sugar chains to also be in the N-conformation from the viewpoint of higher affinity with RNA. Modified nucleic acids such as LNA (Locked Nucleic Acid; 2'-O,4'-C-methylene-bridged nucleic acid (2',4'-BNA/LNA)) have been developed based on this concept. In LNA, for example, by bridging the 2'-position oxygen and 4'-position carbon with a methylene group, the N-conformation becomes fixed and does not fluctuate. Oligonucleotides synthesized by incorporating several LNA units therefore have very high bonding strength and sequence specificity for RNA compared to oligonucleotides synthesized with conventional natural nucleic acid, and exhibit excellent heat resistance and nuclease resistance. Other artificial nucleic acids also have similar characteristics and can therefore be utilized for the present invention. For the purpose of the invention, the 2',4'-modified nucleic acid is preferably LNA.

As mentioned above, the number of 2'-modified nucleic acids in the wing regions is not limited so long as it is a number allowing the toxicity of the bridged antisense nucleic acid to be reduced, and it may be 1 to 10 bases, preferably 1 to 5 bases and more preferably 1 to 3 bases.

According to the invention, the 2'-modified nucleic acid to be added and/or inserted into the wing regions is not restricted and may have the following structural formula: 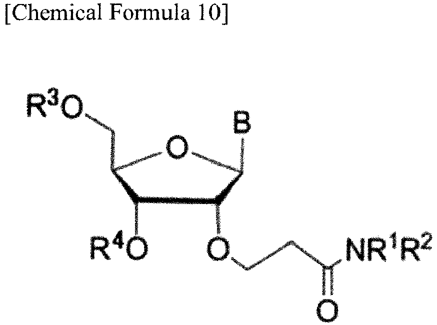 [wherein
R¹ and R² are each independently selected from the group consisting of H, substituted or unsubstituted alkyl groups, substituted or unsubstituted aralkyl groups, substituted or unsubstituted alkenyl groups, substituted or unsubstituted alkynyl groups and substituted or unsubstituted aryl groups;
R³ is H or the structure: 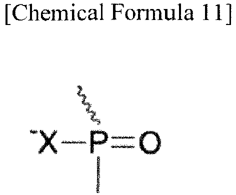 (wherein 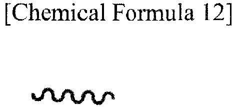 is the bonding point with the adjacent nucleic acid, or OH; and
   X is S or O);
   R⁴ is H or the bonding point with the adjacent nucleic acid; and
   B represents a nucleobase residue optionally having a protecting group or a modifying group]. The term "alkyl group" used here refers to a straight-chain or branched-chain saturated aliphatic hydrocarbon group of 1 to 20 carbon atoms, examples of which include methyl, ethyl, propyl, isopropyl, butyl, isobutyl, *sec*-butyl, *tert*-butyl and cyclohexyl groups. The term "aralkyl group" refers to an alkyl group further substituted with an aryl group, examples of which include benzyl and diphenylmethyl groups. The term "alkenyl group" refers to a straight-chain or branched-chain unsaturated aliphatic hydrocarbon group of 2 to 20 carbon atoms containing 1 to 3 carbon-carbon double bonds, examples of which include allyl and homoallyl groups. The term "alkynyl group" refers to a straight-chain or branched-chain unsaturated aliphatic hydrocarbon group of 2 to 20 carbon atoms containing 1 to 3 carbon-carbon triple bonds, an example of which is propargyl. The term "aryl group" refers to a monovalent residue derived from a compound containing benzene or a structure with two or more benzenes fused or bonded together, or a derivative of the same, and examples include phenyl and naphthyl groups. The term "alkyl group with a substituent" refers to any of the aforementioned alkyl groups further substituted with an amino, halogen, acyl, cyano, alkyl, alkenyl, alkynyl, aryl, aralkyl, alkoxy, alkylthio, acylamino, carbamoyl, carboxy or acyloxy group, examples of which include 2,2,2-trifluoroethyl, cyanomethyl, 2,2,2-trichloroethyl, methoxypropyl and methylthiopropyl groups. The term "aralkyl group with a substituent" refers to any of the aforementioned aralkyl groups further substituted with an amino, halogen, acyl, cyano, alkyl, alkenyl, alkynyl, aryl, aralkyl, alkoxy, alkylthio, acylamino, carbamoyl, carboxy or acyloxy group, examples of which include 4-chlorobenzyl, 4-nitrobenzyl and 2-nitrobenzyl groups. An "alkenyl group with a substituent" is a dichloroallyl group, for example. An "aryl group with a substituent" is a 4-nitrophenyl group, for example.

According to one embodiment of the invention, modified nucleic acid is preferred, wherein the substituent R¹ in the structural formula is preferably H and R² is a methyl group. The present inventors have previously reported that an RNase H-dependent antisense oligonucleotide with introduction of this modified nucleic acid, i.e. 2'-O-(2-N-methylcarbamoylethyl) (MCE) nucleic acid, has lowered hepatotoxicity compared to an antisense oligonucleotide with conventional 2'-O-methyloxyethyl (MOE) modification (Masaki, Y., et al., Nucleic Acid Therapeutics, 28, 307-311 (2018)). It should be noted that the antisense oligonucleotide used is entirely MCE or entirely MOE as the modified nucleic acid in the wing region, while including no bridged nucleic acid such as LNA, and it therefore differs from the present invention.

The bridged antisense nucleic acid of the invention has 2'-modified nucleic acid (typically MCE) supplementally added and/or inserted in the wing region, in a number that is not limited but may be 1 to 8, such as 1 to 5, 1 to 4, 1 to 3, 2 to 5, 2 to 4, 3 to 5, 6, 5, 4, 3, 2 or 1. When 2'-modified nucleic acid is to be added, the positions are at both ends of the wing regions comprising 2',4'-modified nucleic acid, and it may also be introduced into the gap region by substitution of deoxyribonucleotide residues into the gap region. When 2'-modified nucleic acid is to be inserted, it may be within either of the 2',4'-modified nucleic acids. The nucleic acid sequences of the 5'-end wing region and 3'-end wing region after addition and/or insertion of the 2'-modified nucleic acid may be the same or different.

The bridged antisense nucleic acid of the invention can be easily and routinely produced by known technology for solid phase synthesis. For example, it may be carried out by synthesis using the phosphoroamidite method reported by Masaki et al. for MCE-introduced RNase H-dependent antisense oligonucleotide (ibid). Alternatively, it may be carried out by liquid phase synthesis using the modified H-phosphonate method described by Reese et al. (Colin B. Reese and Hongbin Yan, J. Chem. Soc., Perkin Trans. 1, 2002, 2619-2633), or the same method applied to solid phase synthesis. The bridged antisense nucleic acid of the invention is synthesized *in vitro,* and contains no antisense compositions from biological sources or gene vector constructs designed to direct *in vivo* synthesis of antisense molecules. The molecules of the invention may also be mixed, encapsulated, conjugated or bonded with other molecules, molecular structures or compound mixtures, such as liposomes, receptor-targeting molecules, or oral, rectal, local or other types of formulations, in order to facilitate their uptake, distribution and/or absorption.

By having at least one 2'-modified nucleic acid supplementally added and/or inserted in the wing regions, the bridged antisense nucleic acid of the invention has reduced hepatotoxicity compared to the antisense oligonucleic acid before modification (see Example 2). The term "reduced" means that the numerical values of markers used as indicators of hepatotoxicity after administration of a bridged antisense nucleic acid of the invention are lower compared to the antisense oligonucleic acid before modification. For example, the serum concentrations of the hepatotoxicity markers ALT (alanine aminotransferase), AST (aspartic acid aminotransferase) and LDH (lactic acid dehydrogenase) after administration of bridged antisense nucleic acid are preferably 60% or lower, such as 50% or lower, 40% or lower, 30% or lower, 20% or lower, 10% or lower or 5% or lower, with respect to the serum concentrations measured under the same conditions but using the antisense oligonucleic acid before modification.

### (2) Therapeutic agent

As has been demonstrated in Example 2 described below, the bridged antisense nucleic acid of the invention has reduced toxicity while also maintaining activation as an antisense nucleic acid. According to the invention it is possible to provide therapeutic agents (drugs, formulations or medical compositions) similar to commonly used antisense nucleic acids. When the bridged antisense nucleic acid of the invention is to be used as a therapeutic agent, there is no particular limitation on the type of genetic disease. Since the "deoxyribonucleic acid" forming the gap region incorporated into the bridged antisense nucleic acid can target non-coding RNA present in the body (such as microRNA, ribosomal RNA and tRNA), mRNA and single-stranded DNA, antisense nucleic acid containing single-stranded nucleic acid consisting of nucleotide sequences either fully or sufficiently complementary to the nucleotide sequences of the target nucleic acids, where the total or partial sequences of the aforementioned targets are the "target nucleic acid", may be used as therapeutic agents for treatment and/or prevention of genetic diseases corresponding to the target nucleic acids. The length of the deoxyribonucleic acid is generally suitable at about 10 bases, but it is not particularly restricted so long as it is in the range of 5 to 15 bases. The length is preferably 8 to 10 bases and more preferably 10 bases.

Throughout the present specification, the phrase "consisting of a nucleotide sequence (single-stranded nucleic acid) that is sufficiently complementary (to the nucleotide sequence of a target nucleic acid)" encompasses nucleotide sequences that can form base pairs with ≥50% and <100%, preferably ≥60% and <100%, more preferably ≥70% and <100%, even more preferably ≥ 80% and <100% and yet more preferably ≥90% and <100% of the nucleotide sequences of the target nucleic acid. More specifically, this includes cases where, as a result of substituting 1 or 2 to 4 bases with other bases in a nucleic acid chain consisting of a nucleotide sequence completely complementary to the nucleotide sequence of a target nucleic acid, for example, the nucleotide residues can no longer pair at the substituted positions (in which case the positions substituted with other bases are referred to as "mismatched positions"), and cases where, as a result of deleting 1 or 2 to 4 bases in the nucleic acid chain consisting of the nucleotide sequence completely complementary to the nucleotide sequence of the target nucleic acid, for example, the nucleotide residues can no longer pair at the deleted positions.

When the bridged antisense nucleic acid of the invention is to be used as a medical composition, it may also contain another bridged antisense nucleic acid as an active ingredient, as well as medically acceptable carriers, diluents or excipients. The term "medically acceptable" means that the molecular entity and composition is physiologically tolerable and does not produce an allergic reaction or similar harmful reaction, such as acute gastric peristalsis, when administered to a patient. The term "carrier" refers to a diluent, adjuvant, excipient or vehicle administered together with a compound. Such drug carriers may be inert liquids, examples of which are oils containing oils from petroleum, animal, plant or synthetic sources, such as peanut oil, soybean oil, mineral oil or sesame oil, or water. Water or physiological saline and aqueous dextrose and glycerol solutions are preferred as carriers, especially in the case of injections.

As more concrete embodiments of the invention, medical compositions are provided containing bridged antisense nucleic acids in therapeutically effective doses, together with medically acceptable diluents, preservatives, solubilizers, emulsifiers, adjuvants and/or carriers. The compositions also contain additives including various buffers (such as Tris-HCl, acetate or phosphate), pH and ionic strength diluents, detergents, solubilizers (such as Tween80 or polysorbate 80), antioxidants (such as ascorbic acid or sodium metabisulfite), preservatives (such as thimerosal and benzyl alcohol) and bulk substances (such as lactose and mannitol). A medical composition may be prepared in liquid form or as a dry powder such as a lyophilized form.

The medical compositions provided according to the invention are not restricted but are preferably administered by injection, orally, or through the lungs or nose. They are more preferably delivered by an intravenous, intraarterial, intraperitoneal, intramuscular or subcutaneous route of administration.

### (3) Kit

The present invention provides a kit for treatment and/or preventive intervention for a patient with a genetic disease, the kit comprising at least one bridged antisense nucleic acid packaged in a suitable container, and an instruction manual for its use.

The kit contents may be freeze-dried, and the kit may further include a solvent suited for reconstitution of the freeze-dried components. The individual components of the kit may be packaged in separate containers, and may have written information included with the containers, being information of a form established by the government agency regulating the production, use and sale of drugs or biological products, and reflecting approval by institutions regulating their production, use and sale for administration to humans.

When a kit component is provided as one or more liquid solutions, the liquid solution(s) may be aqueous solutions such as sterile water solutions. For *in vivo* use, the expression construct may be formulated into a medically acceptable composition for injection. In this case, the container may be a simple inhalator, injector, pipette, eye drop container or similar apparatus, or the formulation may be applied to the affected area of an animal or injected into an animal, or it may be applied to or mixed with other components in the kit.

The kit components may also be provided in dry form or freeze-dried form. When a reagent or component is provided in dry form, it is generally reconstituted by addition of a suitable solvent for use. In some cases, the solvent may optionally be provided in a separate container. Regardless of the number and types of containers, the kit of the invention may either include, or be packaged with, containers to aid in injection, administration or placement of final complex compositions into animal bodies. Such containers may be inhalators, injectors, pipettes, pincettes, measuring spoons, eye drop containers or other optional medically approved delivery vehicles.

The present invention will now be explained in more specific detail through the following examples, with the understanding that the invention is in no way limited by the examples.

### EXAMPLES

### Example 1: Synthesis of bridged antisense nucleic acid

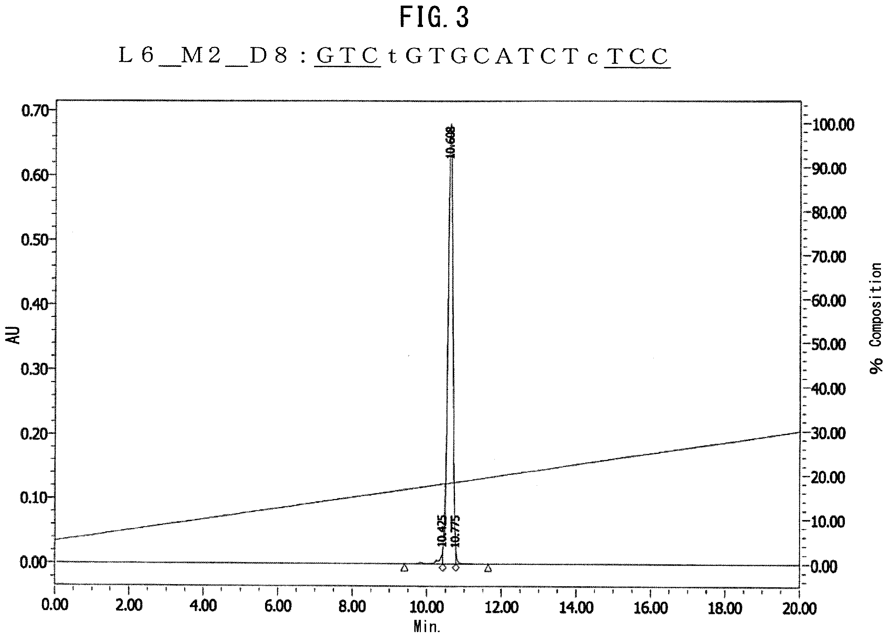

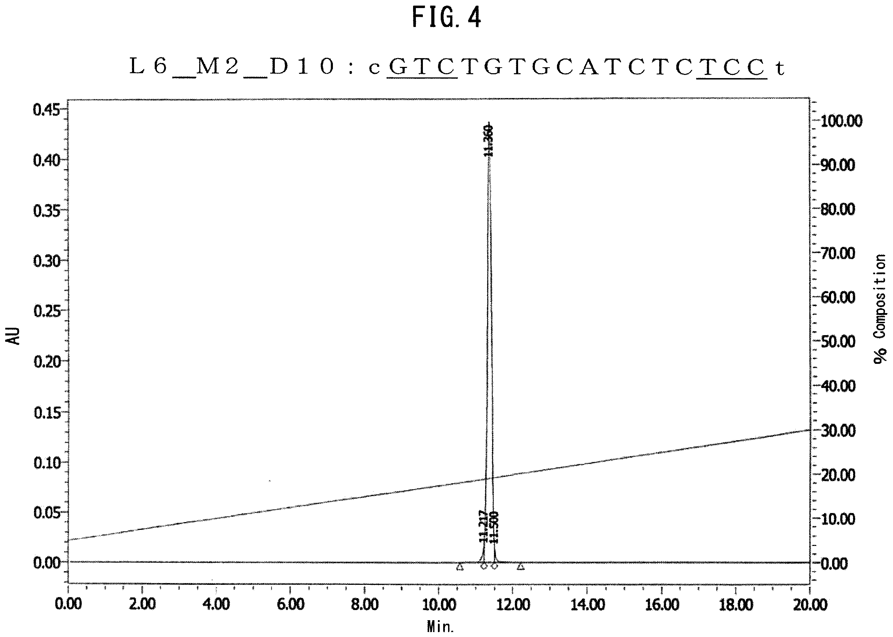

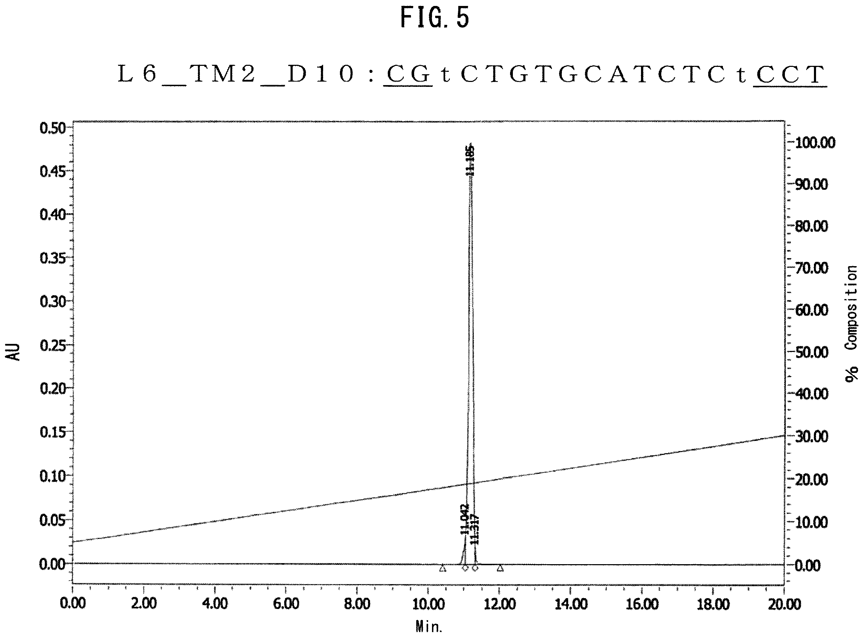

The bridged antisense nucleic acid shown in Figs. 1 to 6, having factor XI as the target gene, were synthesized in a DNA automatic synthesizer by solid phase synthesis using the phosphoroamidite method which is known to those skilled in the art, and were purified and analyzed by reverse-phase HPLC. The underlined portions represent LNA, the alphabetic uppercase represents DNA, and the alphabetic lowercase represents MCE nucleic acid. All of the nucleic acids were linked by phosphorothioate bonds.

L6_D10: GTCTGTGCATCTCTCC (SEQ ID NO: 1)
L4_M2_D10: GTcTGTGCATCTCtCC (SEQ ID NO: 2)
L6_M2_D8: GTCtGTGCATCTcTCC (SEQ ID NO: 3)
L6_M2_D10: cGTCTGTGCATCTCTCCt (SEQ ID NO: 4)
L6_TM2_D10: CGtCTGTGCATCTCtCCT (SEQ ID NO: 5)
L4_M2_D10_mid: GtCTGTGCATCTCTcC (SEQ ID NO: 6)
L5_M1_D10.5: GTcTGTGCATCTCTCC (SEQ ID NO: 7)
L6_M1_D9_nat: GTCTgTGCATCTCTCC (SEQ ID NO: 8)
L6_M2_D10_mm: tGTCTGTGCATCTCTCCc (SEQ ID NO: 9)
Naming of the bridged antisense nucleic acids is "L (LNA)", "D (factor XI gene deoxyribonucleic acid)" or "M (MCE)", with the number of each. For example, "L4_M2_D10" indicates a structure of 4 LNAs, 2 MCEs and 10 DNAs, the nucleic acid conformation being as described above.

### Example 2: Evaluation of bridged antisense nucleic acid hepatotoxicity and activity

### (1) Administration and feed sampling

A physiological saline solution of bridged antisense nucleic acid (3 mg/mL) was subcutaneously administered to 5-week-old female C57BL/6 JJcl mice at 10 mL per 1 kg body weight. After 72 hours, all of the mice were anesthetized with isoflurane and a 23G injection needle and syringe containing 10,000 unit/10 mL of heparin sodium were used for heart blood sampling, from which the blood plasma was harvested. The mice were bled to death by incision in the ventral aorta, and the livers were sampled. The livers were partially trimmed.

### (2) Quantitative PCR analysis of target gene expression

TRIzol reagent (Product No.: 15596018 by Invitrogen) was used for homogenizing of the sampled livers, and the Total RNA was extracted. A portion of the prepared samples was subjected to DNase treatment and purification using a RNeasy Mini Kit (Product No.: 74104 by Qiagen). A 1 st Strand cDNA Synthesis Kit for RT-PCR (AMV) (Product No.: 11483188001 by Roche) was used for cDNA synthesis. Synthesis was in a 20 µL reaction system using Oligo-p(dT)₁₅ primer. Following cDNA synthesis, 20 µL of sterilized water was added for a total of 40 µL. A LightCycler FastStart DNA Master SYBR Green I (Product No.: 12239264001 by Roche) was used for quantitation of the mRNA of each gene.

### (3) Measurement of ALT, AST and LDH

After administering the bridged antisense nucleic acid, the serum concentrations of ALT (alanine aminotransferase), AST (aspartic acid aminotransferase) and LDH (lactic acid dehydrogenase), serving as hepatotoxicity markers, were measured by the JSCC standardized method. As seen by the results in Table 1 below, L6_D10 which did not have MCE introduced exhibited high values for ALT, AST and LDH, but the other bridged antisense nucleic acids containing MCE had low ALT, AST and LDH. Upon typical comparison using the average AST values, the values for L4_M2_D10, L6_M2_D8, L6_M2_D10 and L6_TM2_D10 were 0.03 times, 0.03 times, 0.04 times and 0.06 times L6_D10, respectively, indicating reduction in toxicity on the order of about 100-fold. For FXI/GAPDH (%) as a marker of antisense activity, on the other hand, the values for L4_M2_D10, L6_M2_D8, L6_M2_D10 and L6_TM2_D10 were only reductions at 4.5 times, 8 times, 2.5 times and 2 times L6_D10, respectively, clearly showing reduction in hepatotoxicity with maintenance of antisense activity.

### [Table 1]

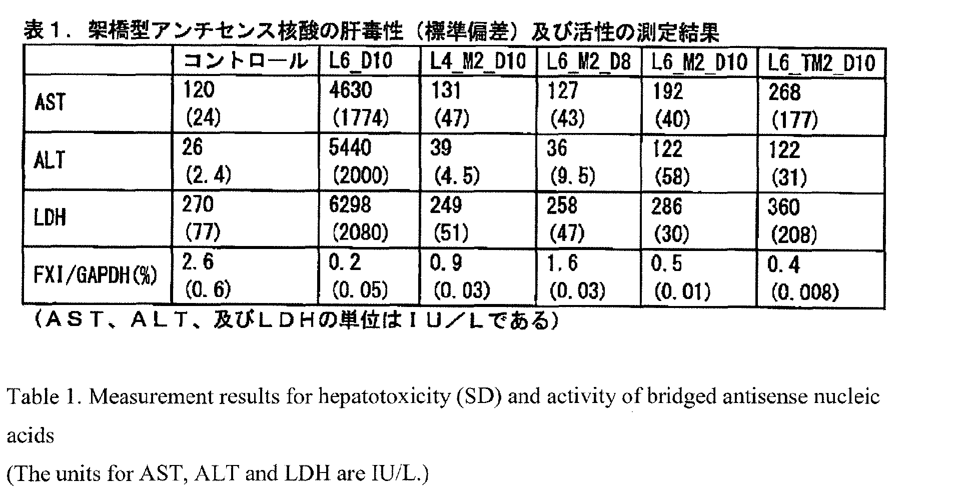

The same experiment was also carried out separately for the four synthesized bridged antisense nucleic acids. The hepatotoxicity and activity of these bridged antisense nucleic acids were measured in terms of AST and ALT.

### [Table 2]

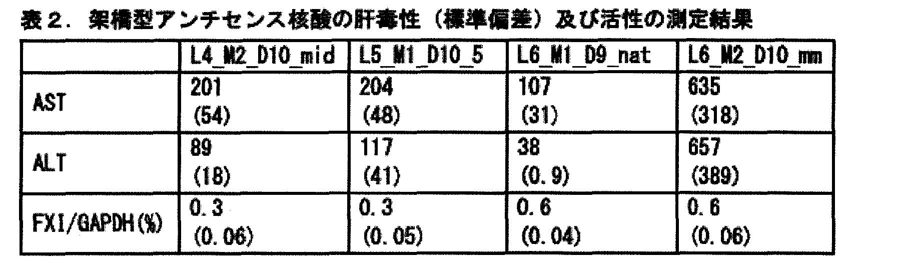

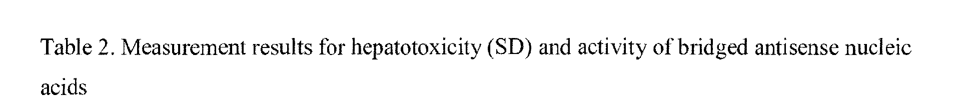

By comparing Table 1 and Table 2 it is seen that the L6_D10 in Table 1, which did not have MCE introduced, exhibited high values for ALT and AST, but the other bridged antisense nucleic acids containing MCE had low ALT, AST and LDH. Upon typical comparison using the average AST values, the values for L4_M2_D10_mid, L5_M1_D10_5, L6_M1_D9_nat, L6_M2 D10_mm were 0.04 times, 0.04 times, 0.02 times and 0.13 times L6_D10, respectively, indicating reduction in toxicity on the order of about 10- to 100-fold. For FXI/GAPDH (%) as a marker of antisense activity, on the other hand, the values for L4_M2_D10_mid, L5_M1_D10_5, L6 M1_D9_nat, L6_M2_D10_mm were only reductions at 1.5 times, 1.5 times, 3 times and 3 times L6 D10, respectively, clearly showing reduction in hepatotoxicity with maintenance of antisense activity.

### INDUSTRIAL APPLICABILITY

Since the bridged antisense nucleic acid of the invention can markedly reduce hepatotoxicity while maintaining its activity, it has very high industrial utility value for providing highly practical antisense nucleic acid drugs.

All of the publications and patent literature cited herein are incorporated into the present specification in their entirety as reference. The specific embodiments of the invention were explained in the present specification for the purpose of example, and it will be readily appreciated by a person skilled in the art that various modifications may be employed such as are not outside of the spirit and scope of the invention.

## Claims

1. Bridged antisense nucleic acid comprising a gap region consisting of deoxyribonucleic acid of 5 to 15 bases and a wing region consisting of two to ten 2',4'-modified nucleic acids at each of the 5' and 3'-ends of the gap region, wherein one to four 2'-modified nucleic acids are supplementally added and/or inserted in at least one wing region.

2. The bridged antisense nucleic acid according to claim 1, wherein the 2'-modified nucleic acid has the following structural formula: 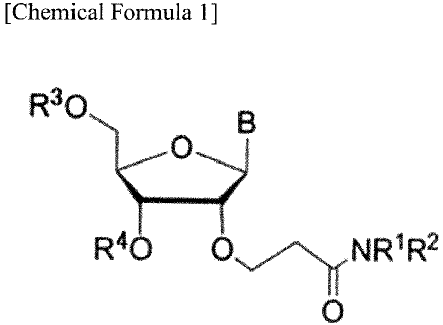 [wherein
R¹ and R² are each independently selected from the group consisting of H, substituted or unsubstituted alkyl groups, substituted or unsubstituted aralkyl groups, substituted or unsubstituted alkenyl groups, substituted or unsubstituted alkynyl groups and substituted or unsubstituted aryl groups;
R³ is H or the structure: 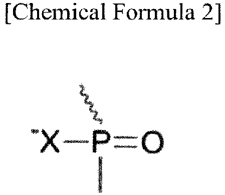 (wherein 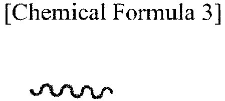 is the bonding point with the adjacent nucleic acid, or OH; and
X is S or O);
R⁴ is H or the bonding point with the adjacent nucleic acid; and
B represents a nucleobase residue optionally having a protecting group or a modifying group].

3. The bridged antisense nucleic acid according to claim 2, wherein R¹ is H and R² is a methyl group.

4. The bridged antisense nucleic acid according to any one of claims 1 to 3, wherein one or two 2'-modified nucleic acids are added and/or inserted in each wing region.

5. The bridged antisense nucleic acid according to any one of claims 1 to 4, wherein the 2',4'-modified nucleic acid is selected from the group consisting of the following: 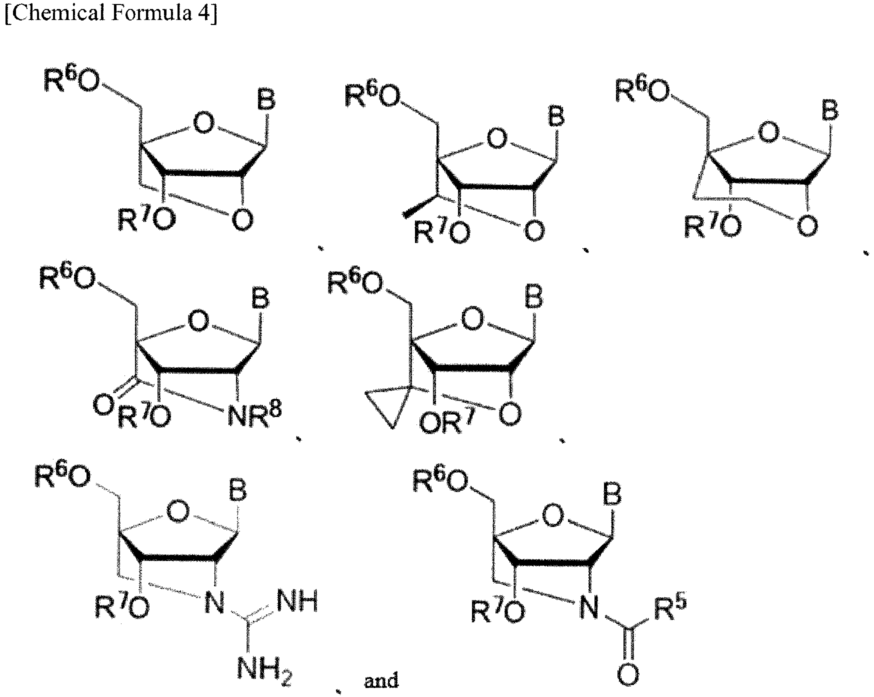 [wherein
R⁵ and R⁸ are each independently selected from the group consisting of H, substituted or unsubstituted alkyl groups, substituted or unsubstituted aralkyl groups, substituted or unsubstituted alkenyl groups, substituted or unsubstituted alkynyl groups and substituted or unsubstituted aryl groups;
R⁶ is H or the structure: 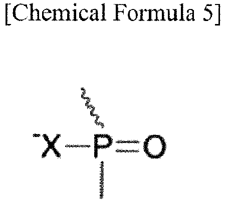 (wherein 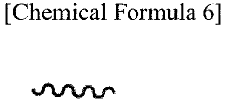 is the bonding point with the adjacent nucleic acid, or OH; and
X is S or O);
R⁷ is H or the bonding point with the adjacent nucleic acid; and
B represents a nucleobase residue optionally having a protecting group or a modifying group] .

6. The bridged antisense nucleic acid according to any one of claims 1 to 5, which comprises one to four 2',4'-modified nucleic acids.

7. The bridged antisense nucleic acid according to any one of claims 1 to 6, wherein X is a sulfur atom.

8. The bridged antisense nucleic acid according to any one of claims 1 to 7, wherein the deoxyribonucleic acid has a base length of 8 to 10.

9. An antisense nucleic acid drug with reduced toxicity by antisense nucleic acid, comprising bridged antisense nucleic acid according to any one of claims 1 to 8.


### [Sequence Listing]

SEQUENCE LISTING

<110> National University Corporation, Tokyo Institute of Technology

<120> Method for reducing the toxicity of antisense nucleic acids

<130> P200667WO

<150> JP2019-214766
<151> 2019-11-27

<160> 9

<210> 1
<211> 16
<212> DNA
<213> Artificial Sequence

<220>
<223> L6 D10

<220>
<221> misc feature
<222> (1)..(3)
<223> locked nucleic acid

<220>
<221> misc feature
<222> (14)..(16)
<223> locked nucleic acid

<400> 1
gtctgtgcat ctctcc                                                    16

<210> 2
<211> 16
<212> DNA
<213> Artificial Sequence

<220>
<223> L4 M2 D10

<220>
<221> misc feature
<222> (1)..(2)
<223> locked nucleic acid

<220>
<221> misc feature
<222> (3)..(3)
<223> 2'-modified nucleic acid

<220>
<221> misc feature
<222> (14)..(14)
<223> 2'-modified nucleic acid

<220>
<221> misc feature
<222> (15)..(16)
<223> locked nucleic acid

<400> 2

gtctgtgcat ctctcc                                                    16

<210> 3
<211> 16
<212> DNA
<213> Artificial Sequence

<220>
<223> L6 M2 D8

<220>
<221> misc feature
<222> (1)..(3)
<223> locked nucleic acid

<220>
<221> misc feature
<222> (4)..(4)
<223> 2'-modified nucleic acid

<220>
<221> misc feature
<222> (13)..(13)
<223> 2'-modified nucleic acid

<220>
<221> misc feature
<222> (14)..(16)
<223> locked nucleic acid

<400> 3
gtctgtgcat ctctcc                                                    16

<210> 4
<211> 18
<212> DNA
<213> Artificial Sequence

<220>
<223> L6 M2 D10

<220>
<221> misc feature
<222> (1)..(1)
<223> 2'-modified nucleic acid

<220>
<221> misc feature
<222> (2)..(4)
<223> locked nucleic acid

<220>
<221> misc feature
<222> (15)..(17)
<223> locked nucleic acid

<220>
<221> misc feature
<222> (18)..(18)
<223> 2'-modified nucleic acid

<400> 4
cgtctgtgca tctctcct                                                  18

<210> 5
<211> 18
<212> DNA
<213> Artificial Sequence

<220>
<223> L6 TM2 D10

<220>
<221> misc feature
<222> (1)..(2)
<223> locked nucleic acid

<220>
<221> misc feature
<222> (3)..(3)
<223> 2'-modified nucleic acid

<220>
<221> misc feature
<222> (15)..(15)
<223> 2'-modified nucleic acid

<220>
<221> misc feature
<222> (16)..(18)
<223> locked nucleic acid

<400> 5
cgtctgtgca tctctcct                                                    18

<210> 6
<211> 16
<212> DNA
<213> Artificial Sequence

<220>
<223> L4 M2 D10 mid

<220>
<221> misc feature
<222> (1)..(1)
<223> locked nucleic acid

<220>
<221> misc feature
<222> (2)..(2)
<223> 2'-modified nucleic acid

<220>
<221> misc feature
<222> (3)..(3)
<223> locked nucleic acid

<220>
<221> misc feature
<222> (14)..(14)
<223> locked nucleic acid

<220>
<221> misc feature
<222> (15)..(15)

<223> 2'-modified nucleic acid

<220>
<221> misc feature
<222> (16)..(16)
<223> locked nucleic acid

<400> 6
gtctgtgcat ctctcc                                                        16

<210> 7
<211> 16
<212> DNA
<213> Artificial Sequence

<220>
<223> L5 M1 D10 5

<220>
<221> misc feature
<222> (1)..(2)
<223> locked nucleic acid

<220>
<221> misc feature
<222> (3)..(3)
<223> 2'-modified nucleic acid

<220>
<221> misc feature
<222> (14)..(16)
<223> 2'-modified nucleic acid

<400> 7
gtctgtgcat ctctcc                                                        16

<210> 8
<211> 16
<212> DNA
<213> Artificial Sequence

<220>
<223> L6 M1 D9 nat

<220>
<221> misc feature
<222> (1)..(3)
<223> locked nucleic acid

<220>
<221> misc feature
<222> (5)..(5)
<223> 2'-modified nucleic acid

<220>
<221> misc feature
<222> (14)..(18)
<223> locked nucleic acid

<400> 8
gtctgtgcat ctctcc                                                        16

<210> 9

<211> 18
<212> DNA
<213> Artificial Sequence

<220>
<223> L6 M2 D10 mm

<220>
<221> misc feature
<222> (1)..(1)
<223> 2′-modified nucleic acid

<220>
<221> misc feature
<222> (2)..(4)
<223> locked nucleic acid

<220>
<221> misc feature
<222> (15)..(17)
<223> locked nucleic acid

<220>
<221> misc feature
<222> (18)..(18)
<223> 2′-modified nucleic acid

<400> 9
tgtctgtgca tctctccc                                                    18